Europäisches Patentamt

European Patent Office

Office européen des brevets

(19)

(11) Publication number: **0 356 130**
**A2**

# EUROPEAN PATENT APPLICATION

(12)

(21) Application number: **89308323.8**

(22) Date of filing: **16.08.89**

(51) Int. Cl.⁵: **C 12 N 15/70**

(30) Priority: **22.08.88 US 234727**

(43) Date of publication of application:
**28.02.90 Bulletin 90/09**

(84) Designated Contracting States: **DE FR GB**

(71) Applicant: **EASTMAN KODAK COMPANY**
**343 State Street**
**Rochester New York 14650 (US)**

(72) Inventor: **Weber, Shane Crawford c/o EASTMAN KODAK**
**COMPANY**
**Patent Department 343 State Street**
**Rochester New York 14650 (US)**

**Holzschu, Donald Lee c/o EASTMAN KODAK COMPANY**
**Patent Department 343 State Street**
**Rochester New York 14650 (US)**

**Lalik, Patricia Helen c/o EASTMAN KODAK COMPANY**
**Patent Department 343 State Street**
**Rochester New York 14650 (US)**

(74) Representative: **Nunney, Ronald Frederick Adolphe et al**
**Kodak Limited Patent Department Headstone Drive**
**Harrow Middlesex HA1 4TY (GB)**

(54) **A mobile f1 phage single-strand DNA origin of replication.**

(57) A f1 phage intergenic region having the same synthetic restriction site on each of its flanks is mobile. It can be used to conveniently convert plasmids to phagemids.

FIG. I

**Description**

## A MOBILE f1 PHAGE SINGLE-STRAND DNA ORIGIN OF REPLICATION

### II. Field of the Invention

This invention relates to the field of genetic engineering; to a novel tool for use in the field and to methods of making and using the tool.

### III. BACKGROUND OF THE INVENTION

The f1 phage intergenic region f1( IG) of bacteriophages contains the origin of replication for synthesis and packaging of phage single-stranded DNA. This region, when incorporated into a plasmid that contains only an origin of replication for double-stranded DNA, such as a ColE1 replicon, converts the plasmid to a phagemid. A phagemid is a DNA molecule that can exist either in a single-stranded form like a phage, or as a double-stranded DNA like a plasmid depending upon whether the plasmid or phage origin of replication is active. Genetic engineering is often conducted on single-stranded DNA.

A protein expression vector is designed to over-express a protein so that adequate amounts of the protein are available. The protein may be a normal protein, a mutant protein or an entirely new protein. A protein expression vector may include, in operative association, such genetic functional regions, inter alia, as promoters, regulatory regions for the promoter, a repressor gene for a repressor of the promoter, an RNA polymerase gene for the polymerase used by the promoter, enhancers of transcription, 5' messenger RNA sequences for high efficiency translation, 3' stabilizers of messenger RNA half life, transcription termination regions and polyadenylation sites for the messenger RNA.

Protein engineering involves protein structure modifications to change protein function. The desired modifications are accomplished by changing the underlying DNA base sequence of the gene that codes for a given protein. Generally this is done by changing a few bases at a time.

Protein engineering is generally done in a two step process beginning with single-stranded DNA. First, in sequencing and mutagenesis vectors, the DNA for a given gene is mutated to cause a change in the amino acid sequence of the protein expressed by that gene. Second, the mutated gene or DNA fragment is then transferred to a protein expression vector that causes a mutant protein to be over-expressed.

### IV. Problems of the Prior Art

There are a number of conceptual and technical limitations to the above approach to protein engineering which restrict protein engineering particularly in the design of protein chimeras in which protein exon folding domains derived from different genes are mixed.

The approach of mutagenesis followed by transfer of the mutated gene to an expression vector is time consuming and labor intensive. Moreover, the ability to precisely work with DNA nucleotides is extremely limited because transfer of the gene to an expression vector occurs through restriction endonuclease technology.

Restriction endonucleases catalyze the hydrolysis of certain phosphodiester bonds of DNA, at specific DNA sequences frequently referred to as restriction sites. Different endonucleases recognize different nucleotide sequences. The recognition sequences are randomly dispersed throughout the DNA of an organism. Shorter recognition sequences occur with higher frequency than longer ones. Due to the random distribution of restriction sequences, the ability to cleave a DNA molecule at a desired locus, for excision of a segment of DNA, such as a segment corresponding to a discrete protein exon, or for insertion of a new segment, is only possible when a restriction sequence is found at or near the desired locus. This limitation is a severe limitation in protein engineering. Every new project must be handled on an ad hoc basis, depending upon where the restriction sequences are located in each case. Extensive mapping of restriction sequences may even be necessary to determine whether the desired insertions or excisions are feasible. Nucleotide sequence analyses may also be required.

In existing phagemids, such as sequencing and mutagenesis vectors, containing an f1(IG), there are few natural restriction sequences immediately adjacent to each flank of f1(IG). This region is also known as the f1 phage origin of replication in the literature. The few close natural restriction sequences that exist generally form blunt ends when treated with the appropriate enzyme. Blunt ends are inefficient in that they do not ligate easily with other blunt ends, and not with cohesive ends at all. In DNA sequences further removed from f1(IG) many random restriction sites occur, for example in the multiple cloning (polylinker) sites of plasmids. However, use of these sequences to remove f1(IG) results in a long piece of DNA that cannot be inserted into other plasmids without adverse effects due to intraplasmid homologous recombination.

The longer the stretch of DNA comprising f1(IG), the more difficult it is to insert it into the DNA sequence of a plasmid. Longer stretches of DNA are likely to include other restriction sites further complicating vector construction strategies since the strategic choice of enzymes for editing becomes more complicated.

In the field of protein engineering there is a clear and present need to overcome or minimize the limitations imposed on the construction of a wider variety of protein expression vectors or protein produced from such vectors by the limitations of restriction endonuclease technology.

### V. Summary of the Invention

The present invention provides a f1 phage origin of replication having the same synthetic restriction endonuclease site (restriction site) on each of its immediate flanks. It will be sometimes referred to hereinafter as mobile f1. The phrase mobile f1 also includes f1(IG)-bearing mutations that do not adversely affect its ability to convert plasmids to phagemids.

The flanks of the f1(IG) are those DNA base pair sequences, upstream or downstream, on either side of the region. The exact base pair length of the functional f1(IG) has not been established. Workers in the field have used DNA sequences comprising from about 1,300 to 454 base pairs as comprising the region. It is possible that even longer or shorter lengths would be operative in converting plasmids to phagemids. Thus, the base pair length and position of the flanks will vary depending upon the base pair length of the DNA selected as comprising the region. Regardless of the length of mobile f1, the flanks thereof will contain a restriction site on each flank that is not naturally present therein. Such site is synthetic in that it is inserted into the flanks using techniques such as site-directed mutagenesis. It is preferable that the restriction site be as close to f1(IG) as possible without interfering with the capability of f1(IG) to convert plasmids to phagemids.

The phage restriction endonuclease site or restriction site includes the case where the site has been cut with a restriction endonuclease. Thus mobile f1 will generally have the same synthetic restriction endonuclease site on each flank.

## VI. Advantages of Mobile f1

The presence of synthetic restriction sites on each flank of mobile f1 makes it relatively easy to insert mobile f1 into any plasmid. Thus mobile f1 makes possible a wide variety of phagemids that can be isolated in a single-stranded form suitable for further genetic engineering manipulations. These vectors can be used to construct novel proteins by fusing, at the exon DNA level, different protein folding domains.

Typically, the process of gene cloning and protein over-expression involves essentially a cut and paste operation involving several different plasmids to produce a single protein expression vector. Use of mobile f1 to convert plasmids to phagemids makes it possible to perform the following operations in a single plasmid: 1) DNA sequencing to confirm the results of mutagenesis; 2) synthesis of ssDNA probes for Southern analysis of DNA or Northern analysis of messenger RNA structure, expression, plasmid stability; and 3) DNA oligonucleotide directed mutagenesis to a) create convenient restriction sites; b) generate mutant proteins and/or protein chimeras; c) redesign natural or synthetic genes; d) adjust promoter to coding region spacing for protein over-expression; e) alter DNA sequences to construct better contexts for functional units, such as DNA sequences that affect the efficiency of translational initiation; f) insert new DNA sequences in an oligo sequential manner that encode functional regions or even whole genes (150 bases is a typical insertion); g) delete DNA; and h) construct gene fusions involving multiple genes or gene fragments encoding specific protein folding domains.

## VII. Brief Description of the Drawings

Figure 1 is a schematic of the method of making mobile f1.

Figure 2 is the DNA sequence of a specie of mobile f1.

Figure 3 is a schematic of a method for converting a plasmid to a phagemid using mobile f1.

## VIII. Details of the Invention

### A. Introduction

The conversion of any existing plasmid to a phagemid is accomplished by joining mobile f1 to a receiving form of that plasmid. Mobile f1 and the receiving plasmid are separately prepared and then joined by ligation. This produces a phagemid that has the property of f1 single-strand DNA replication and all the previously existing properties and functions of the starting plasmid.

The conversion of plasmids to phagemids with mobile f1 are made using an unobvious combination of various conventional teachings of recombinant DNA technology, particularly as described in Molecular Cloning: A Laboratory Manual, T. Maniatis et al, Cold Spring Harbor; Advanced Bacterial Genetics, R.W. Davis et al, Cold Spring Harbor and Experiments with Gene Fusions, T.J. Silhavy, Cold Spring Harbor.

### B. Method of Mobile f1 Preparation

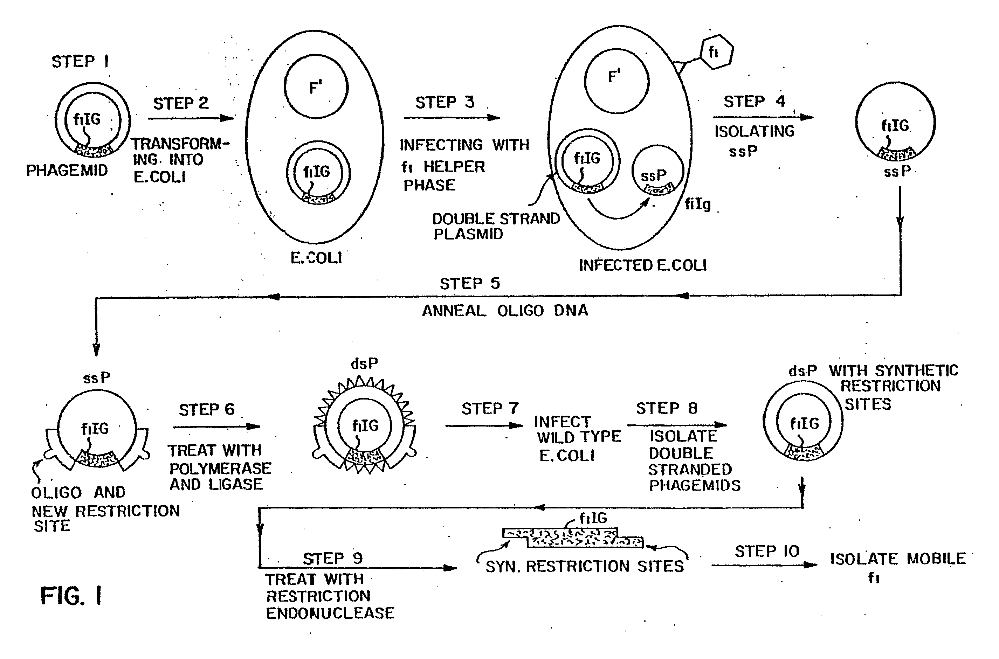

Referring to figure 1, the method of mobile f1 preparation comprises the steps of:

1) providing a phagemid having a f1 phage intergenic region [f1(IG)];

2) transferring the phagemid of 1) into an E.coli strain that

    i) is deficient in dUTPase and uracil N-glycosylase and

    ii) includes a F′ element;

3) infecting E.coli resulting from 2) with a f1 helper phage (f1) thereby forming a single-stranded DNA version of the phagemid of 1) containing the f1 phage intergenic region and uracil bases (ssP);

4) isolating single-stranded phagemid (ssP) containing uracil and f1 phage intergenic region [f1(IG)] secreted by the infected E.coli;

5) annealing on each flank of the f1 intergenic region of the single-stranded phagemid, a mutagenic oligonucleotide (oligo) containing a synthetic restriction site and a 5′ phosphate wherein the restriction site is the same on each flank;

6) treating the phagemid of 5) with DNA polymerase and DNA ligase to synthesize a DNA strand complementary to the plasmid of 5) resulting in a closed double-stranded phagemid;

7) infecting a wild-type E.coli strain with the phagemid of 6) thereby allowing the growth of double-stranded phagemids (dsP) having the synthetic restriction sites on the flanks of the f1 intergenic region and the destruction of phagemids containing uracil bases;

8) isolating the double strand phagemids containing the synthetic restriction sites;

9) treating the double-stranded phagemid (dsP) with a restriction endonuclease that recognizes the synthetic restriction sites, thereby removing the f1 intergenic region from the phagemid;

10) isolating the f1 phage intergenic region having the same synthetic restriction site fragments on each flank, resulting in mobile f1.

### C. An Illustrative Procedure for Making Mobile f1

A general method of making mobile f1, presented in VIII.B. supra, was implemented as follows. Variations on this procedure can be made within the spirit and teachings of the general method.

A phagemid containing a f1 phage intergenic region was selected. Examples of available plasmids include pUCF1 (Pharmacia), Genescribe pTZ18R, pTZ18U, pTZ19R and pTZ19U (United States Biochemical) and pVT100, 101, 102 and 103 (Vernet & Thomas).

The phagemid pTZ18R was selected herein to exemplify an embodiment of the general method of preparing mobile f1. The objective is to modify pTZ18R by inserting, by oligo mutagenesis, the same synthetic restriction site on each flank of the f1 phage intergenic region. A method similar to the procedure of Kunkel, 1985, Kunkel, Roberts and Zakour, 1987, was used for this objective.

E.coli strain CJ236 was transformed with pTZ18R. This strain is deficient in two enzymes:

a) dUTPase (dut) which results in elevated intracellular concentrations of dUTP that competes with dTTP for incorporation into replicated DNA; and

b) uracil N-glycosylase (ung) which removes uracil from DNA. Hence uracil bases are left in plasmids when grown in CJ236.

The genotype of CJ236 is dut 1, ung 1, thi 1, rel AI/pCJ105 in which pCJ105 is a chloramphenicol selectable F′ produced by Catherine Joyce at Yale University.

CJ236 was transformed with pTZ18R by a standard CaCl$_2$ transformation procedure described in IX.B., infra.

A pTZ18R comprising uracil bases from transformed CJ236 was chosen for the preparation of a single strand template by ampicillin resistance selection according to IX.C, infra. CJ236 containing pTZ18R was infected with R408 helper phage (available from Pharmacia-PL Biochemicals, Stratagene or Norton D. Zinder, Rockefeller University) to produce and secrete preferentially single-stranded pTZ18R phagemids containing uracil bases. R408 encodes for the f1 phage proteins necessary for nicking the f1 intergenic region of pTZ18R thereby initiating single-stranded replication of pTZ18R. The f1 phage provides other proteins necessary for the final packaging and secretion of pTZ18R phagemids from CJ236 for use as templates. The preparation and isolation of the templates was carried out according to section IX.C., infra.

Two mutagenic DNA oligonucleotides (oligo) were synthesized that were complementary to each flank of the f1 intergenic region in the uracil-containing pTZ18R packaged above, except that each oligo contained three non-complementary bases of new information. This resulted in a block of six bases in each flank forming a BamHl site.

The sequences of these oligos were:
Upstream 5′GCG-CAG-CCT-GAA-TGG-CGA-ATG-"GGA-TCC"-TGT-AAA-CGT-TAA-TAT-TTT-G3′
Downstream 5′GCG-CCG-CTA-CAG-CGC-GCG-TCA-"GGA-TCC"-ACT-TTT-CGG-G3′

The sequences enclosed in quotation marks, GGA-TCC, are BamHl restriction sites formed from the three base changes contained in the mutagenic oligos. The BamHl sites will flank the F1 intergenic region in phagemids generated from pTZ18R when mutagenesis is completed.

The synthetic restriction site selected must be fusible with, or made fusible with the receiving site sequence of the selected receiving plasmid. Alternatively, the DNA sequence of the receiving plasmid can be made fusible with the selected synthetic restriction site for mobile f1. Such DNA sequences are also restriction sites.

Table I presents illustrative fusible restriction sites.

#### TABLE I

| Exemplary Restriction Site | Is Fusible With |
|---|---|
| BamHl | Bcll |
| | Bglll |
| Sall | Xhol |
| Xbal | Spel |
| | Avrll |
| | NHel |

Each oligo was purified by gel electrophoresis on Tris-borate -EDTA urea acrylamide gels, (Maniatis, Fritsch and Sambrook, 1982). The two purified oligos were phosphorylated at the 5′ end by the use of T4 polynucleotide kinase (Maniatis et al.).

The two phosphorylated full length oligos were annealed to the single strand uracil containing pTZ18R template obtained from the procedure in IX.C. Each flank of f1(IG) was paired with a specific oligo. The annealing reaction was carried out by mixing 1 pmole of single strand template with 1 to 3 pmole of each oligo in a reaction volume of 25 microliters of 10 mM MgCl$_2$, 10 mM Tris, pH 7.5. The mixture was heated at 55°C for 5 minutes. The mixture was then cooled at room temperature for 15 minutes.

Using the template and the oligos as primers, DNA complementary to the template was synthesized with Klenow polymerase. The polymerase added DNA sequences that were complementary to the template up to and between the oligos. Gaps between the oligos and the complementary DNA sequences were ligated with T4 DNA ligase to form a covalent bond between the synthetic oligos and the synthesized complementary strand. This was done by subjecting 25 microliters of the template having the annealed oligos to 25 microliters of a mixture containing 50 μMolar each of dATP, dCTP, dGTP and dTTP, 10 units of Klenow, 10 units of T4 DNA ligase, 10 mM MgCl2, 10 mM Tris (pH 7.5) and 5 mM DTT. The combined mixture was placed in ice for 5 minutes; then maintained at room temperature for 5 minutes and then at 30°C for 2 hours. This reaction mixture now contains a covalently-closed circular (ccc) double-stranded derivative of pTZ18R. One strand contained uracil bases. The complementary strand was composed of the two oligos and newly synthesized DNA containing no uracil bases. This complementary strand has a new BamHI site on each flank of f1(IG). The original strand containing uracil bases has no new BamHI sites.

Wild type E.coli strain TBl was transformed with ccc pTZ18R by the CaCl2 transformation procedure in IX.B., infra. TBl permits replication of thymine containing plasmids. It does not permit replication of uracil containing plasmids. This property of TBl provides for preferential selection and growth of pTZ18R derivative plasmids from the strand containing the mutagenic oligos.

Ampicillin resistant colonies were selected from which transfectants were randomly picked and grown up at 37°C in 5 ml LB + 100 μg ampicillin/ml small scale cultures overnight. Small amounts of each transfectant were prepared by a standard mini DNA preparative procedure, section IX.E., infra.

DNA from each transfectant was analyzed by restriction endonuclease cutting. Screening was performed on 14 transfectants by cutting each with BamHI restriction enzyme for 1.5 hours at 37°C in a 20 microliter reaction mixture consisting of 17 microliters of each transfectant from the mini preparations, 5-20 units of BamHI. Ten μg Ribonuclease A and 20 μl of buffer containing 100 mM NaCl, 10 mM Tris, 10 mM MgCl2 and 20 μg BSA (pH 7.8). A no enzyme (BamHI) negative control was performed concurrent for each sample.

To visualize BamHI fragments, agarose gel electrophoresis was performed according to Maniatis et al at an agarose concentration of 2.0% (w/v) except that the agarose contained 1 μg/mL of ethidium bromide. Gels were generally subjected to electrophoresis at 80-100 volts for 2 to 3 hours at room temperature. The gels were photographed according to Maniatis et al.

From the above analysis one of the double-stranded phagemids containing BamHI oligo mutants was selected and designated, pSCW526. It had a small 455 base pair f1(IG) having synthetic BamHI site on each flank thereof.

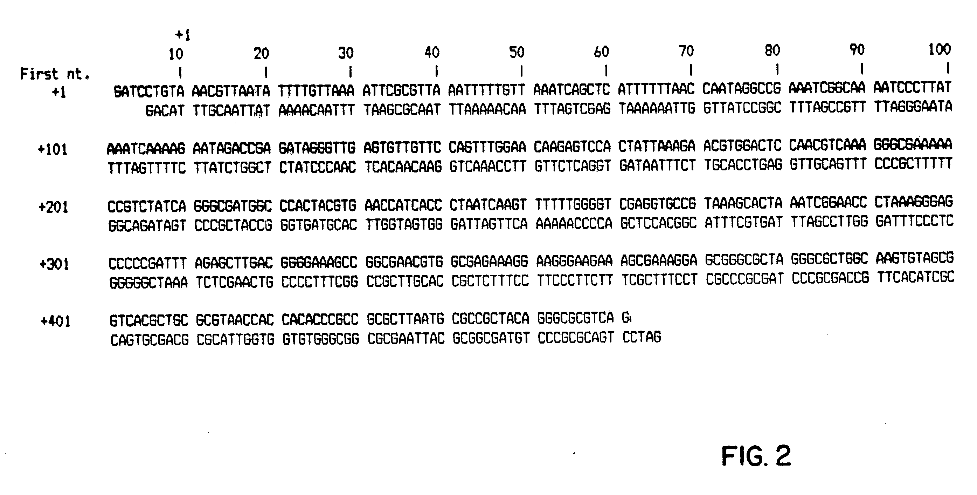

The small 455 base pair sequence was removed from pSCW526 by treatment with BamHI. It was designated BamHI mobile f1, in all further manipulations (figure 2). BamHI mobile f1 is a species of mobile f1.

To prepare useful quantities of BamHI mobile f1, large amounts of pSCW526 were prepared by the standard large scale 600 milliliter plasmid preparative procedure of IX.D., infra.

BamHI mobile f1 was removed from pSCW526 by digesting 20 μg of pSCW526 in 500 μl containing 100 mM NaCl, 10 mM MgCl2, 10 mM Tris (pH 7.8) and 200 units of BamHI. The BamHI digestion proceeded for 4 hours at 37°C. A portion containing about 1 microgram was checked by agarose gel electrophoresis until the reaction was sufficiently complete for purification of BamHI mobile f1.

A preparative Tris-phosphate agarose gel containing ethidium bromide as previously described herein was loaded with the BamHI digested pSCW526 gel sample. This sample was subjected to electrophoresis overnight at 20 volts. The separated DNA fragments in the agarose gel were visualized by short wavelength ultraviolet light. The 455 base pair BamHI mobile f1 was recognized by comparing its mobility to DNA fragment molecular weight markers. The BamHI mobile f1 was cut out in an agarose strip and purified by a standard electroelution procedure in section IX.F., infra.

The purified BamHI mobile f1 with BamHI cohesive ends (figure 2) was confirmed to be free of DNA contaminants by agarose gel electrophoresis. BamHI mobile f1 was ready for insertion into a receiving plasmid.

## D. Method of Making a Phagemid Comprising Mobile f1

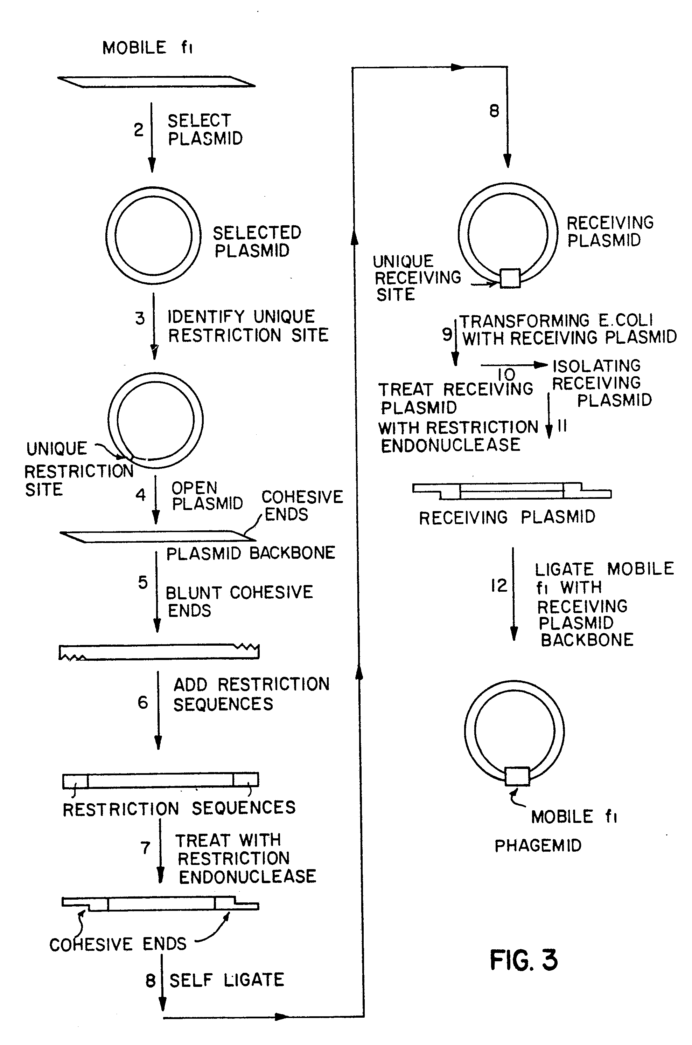

Referring to figure 3, a general method of phagemid preparation comprising mobile f1 comprises the steps of:

1) providing a mobile f1;

2) selecting a plasmid into which mobile f1 is to be inserted;

3) identifying a unique restriction endonuclease site on the plasmid;

4) opening the plasmid at the unique site to form a plasmid backbone;

5) blunting, if necessary, any cohesive ends of the plasmid backbone;

6) adding a preselected restriction sequence to each end of the backbone that will fuse with the synthetic restriction site on each flank of mobile f1;

7) cutting the backbone by treating it with the preselected restriction endonuclease thereby forming cohesive ends on each end of the plasmid backbone;

8) self-ligating the cut backbone of step 7) to form a receiving plasmid having the preselected restriction site thereby forming a unique receiving site;

9) transforming E.coli with the receiving plasmid;

10) isolating the receiving plasmid;

11) treating the receiving plasmid with the preselected restriction endonuclease enzyme to form a receiving plasmid backbone; and

12) forming a phagemid by ligating mobile f1 with the receiving plasmid backbone.

Although the phagemid was completely formed in step 12) above, it was present in extremely small concentrations together with quantities of plasmid DNA that did not form the desired phagemid. Therefore, it was necessary to grow, identify and isolate the phagemid. This was carried out using the techniques presented in section IX., infra. More specifically such techniques were

1) transforming an E.coli strain that contains a f1 element with the mixture comprising the phagemid;

2) identifying and isolating the phagemids by restriction endonuclease and agarose gel electrophoresis analysis;

3) infecting an E.coli containing phagemid with helper f1 phage; and

4) isolating single-stranded DNA phagemid of 3).

### E. Criteria for Selection and Modification of Plasmid to Receive a Mobile f1

The plasmid in which mobile f1 is inserted is selected on the basis of its ultimate use such as sequencing, protein expression, retroviral work, transfection or cloning of genes or their cDNA's.

A unique restriction site in the selected plasmid is chosen as the receiving site for mobile f1. A unique restriction site is a site that appears only once in the plasmid. The receiving site is outside regions 1) required for the plasmid's biological functions, 2) expected to be used by the genetic engineer or 3) otherwise essential to the plasmid.

Ideally the receiving site should also be compatible with the synthetic restriction sites on mobile f1. Of course, the receiving site or the restriction site on mobile f1 can be made compatible with each other through mutagenesis or by treatment with linkers. Compatibility is obtained, for example, when the receiving site and the synthetic restriction sites are recognized by the same restriction endonuclease or have complementary cohesive ends. When this type of compatibility exists, only steps 1-4 and 12 of the general method need be performed to form a phagemid comprising f1(IG).

Compatibility, for the purposes of the present invention, is also obtained when the receiving site and the synthetic restriction site can be fused together into a DNA sequence that is not recognized by restriction endonuclease for either site. When this type of compatibility exists, the entire general method is used.

If necessary, either the receiving site or the synthetic restriction site can, through the use of linkers, be converted to a DNA sequence that fuses with the synthetic restriction sites or the receiving site to form a site that is not recognized by either restriction endonuclease.

Restriction sites are fusible when two different restriction sites are joinable but upon joining do not recreate either of the starting restriction sites. Many restriction endonuclease recognition sites are six base (1 to 6) DNA sequences. When two different endonucleases recognize different sites in which base sequences 2-5 are the same, the sites are fusible. The fused site will not be recognized by either starting restriction endonuclease.

In this approach ligating mobile f1 into a plasmid to form a phagemid results in the immobilization of the f1 single strand DNA origin of replication in the phagemid.

Particularly useful combinations of fusible restriction sites are presented, supra.

Since the synthetic restriction sites of mobile f1 are the same on both flanks, it is possible to insert mobile f1 into the selected plasmid at the receiving site in either direction thereby permitting manipulations of either strand of the resulting phagemid.

### F. An Illustrative Procedure for Preparing Phagemids

The above general procedure for making a phagemid may be carried out as follows. It will be understood that variations on this specific procedure may be conducted within the spirit and teachings of the general procedure. This procedure is a variation of standard techniques cited herein before.

In this procedure BamHI mobile f1 (figure 2) was inserted into a protein expression plasmid, pSCW231, prepared in our labs to make final phagemid pSCW576. Any plasmid could be used.

In pSCW231 the unique restriction site was TthIIII. This site was about 200 base pairs downstream of a double strand origin of replication (Col E1). This restriction site was chosen because it is in a functionally unessential region of pSCW231, but yet very close to the Col E1 origin of replication.

As shown in Table I, BamHI sites are fusible with BglII sites. BglII sites are relatively rare and are not present in many protein expression vectors. The TthIIII site can be converted with DNA linkers to a BglII site.

The resulting fused sequence (BamHI/BglII) will be in a region of the plasmid that will not interfere with subsequent construction therein. For example, a TthIIII site, within 200 base pairs of a plasmid replicon, will almost always be present since most plasmids will not have had deletions this close to this replicon. Therefore insertion of mobile f1 in this innocuous out of the way position will not interfere with other functional regions of the protein expression vector.

pSCW231 was first converted into pSCW480, a receiving plasmid as follows:

Two μg of pSCW231 was cut with 50 units of TthIIII for 2 hours at 65°C in 100 μL containing 50 mM NaCl, 10 mM Tris, pH7.5, 10 mM MgCl$_2$, 1 mM DTT and 100 μg BSA/mL. This resulted in one large linear plasmid backbone having cohesive TthIIII fragments as follows:

5′ GACN NNGTC 3′
3′ CTGNN NCAG 5′

The cohesive ends were blunted by adding to the backbone reaction mixture 10 units of Klenow DNA polymerase; then dATP, dCTP, dGTP and dTTP were each added to a final concentration of 50 μM. The blunting reaction proceeded for 30 minutes at 30°C. The polymerase was inactivated by heat treatment of the mixture at 65°C for 10 minutes.

Other equivalent methods of blunting include 1) use of other polymerases such as DNA polymerase I from E.coli or T4 phage DNA polymerase, 2) exonucleases with either single- or double-stranded specificity or 3) the use of DNA polymerases as a nuclease through use of proofreading nuclease activity in the absence of one or more dNTP's.

The selected receiving site (BglII) was added to the blunted pSCW231 backbone with DNA linkers and T4 DNA ligase as follows. Two μg of phosphory-

lated BglII linkers were added to the blunted pSCW231 mixture along with 10 U's of T4 DNA ligase. ATP was also added to a final concentration of 1 mM. Ligation of the BglII linkers to blunted pSCW231 backbone was carried out at 16°C for 16 hours. The ligase in the reaction mixture was heat inactivated at 65°C for 10 minutes. The BglII sites added to the plasmid backbone were cut by adding to the mixture 100 U's of BglII and maintaining it at 37°C for 4 hours. The plasmid backbone, now having cut BglII sites on each end, was then extracted sequentially with 500 μL of phenol and 500 μL of a isoamylalcohol:chloroform.

Cut BglII linkers were removed from the reaction mixture by ion exchange as follows. NaCl to a concentration of 200 mM was added to the reaction mixture. The mixture was passed through a cation ion exchange column (Maniatis et al). At this NaCl concentration the large plasmid backbone binds to the column. Cut BglII linkers flow through. The plasmid backbone having cohesive BglII restriction ends was removed from the column with 0.8 mls of 2M NaCl in TE. The backbone was precipitated with 2 μg of carrier yeast t-RNA and 150 mM NaCl in ethanol. The precipitated backbone was resuspended in TE. A portion containing 0.5 μg of the backbone was self ligated for 16 hours at 16° with 10 U's of T4 DNA ligase in 25 μl containing 10 mM Tris, pH 7/8, 10 mM MgCl$_2$, 1 mM ATP, 1 mMDTT and 25 μg BSA. The resulting self-ligated backbone was used to transform HB101 with ampicillin selection according to general procedure IX.B., infra.

Transfectants were randomly picked. They were grown up at 37°C in 5 ml of LB + 100 μg ampicillin/ml small scale cultures overnight. Plasmid DNA from each transfectants was prepared by a standard mini DNA preparative procedure in section IX.E., infra. The DNA from each transfectant was analyzed by BglII restriction and agarose gel electrophoresis. A transfectant containing a BglII site at the previous Tth III I site was identified by this analysis and designated pSCW480.

BamHI mobile f1 was inserted into pSCW480 at the BglII site. First, pSCW480 was cut with BglII. Second, BamHI mobile f1 was ligated to pSCW480. These two steps were carried out as follows.

A preparative amount of pSCW480 was prepared according to section IX.C., infra. Ten μg of pSCW480 was cut for 2 hours at 37°C with 100 U's of BglII in 500 μL containing 50 mM NaCl, 10 mM Tris, pH 8.0 and 10 mM MgCl$_2$.

It was preferable to remove the 5' phosphates at this time. This was accomplished by adding 10 U's of calf intestinal phosphatase for 30 minutes at 37°C followed by heat inactivation at 65°C for 10 minutes.

The dephosphorylated pSCW480 backbone (0.25 mg) was added to 0.5 μg of BamHI mobile f1 in a volume of 25 μL containing 10 mM MgCl$_2$, 10 mM Tris, pH 7.8, 1 mM ATP and 25 μg of BSA. The pSCW480 backbone and mobile f1 were joined by ligation with 10 U's of T4 DNA ligase for 16 hours at 16°C.

The resulting phagemid containing a f1(IG) region was designated pSCW576 and transformed into the E.coli strain SCW105. This E.coli strain contains a Kanamycin selective F' marker.

Random transfectants of E.coli strain SCW105 were randomly picked. They were grown up at 37°C in 5 mL LB containing 100 μg ampicillin/ml. Plasmids were prepared by a standard mini-DNA preparative procedure, section IX.E. Plasmids from each transfectant were analyzed by restriction endonuclease cutting to identify plasmids in which the successful joining of BamHI mobile f1 to the BglII receiving site in pSCW480 had occurred. Based on this analysis, one transfectant was designated as phagemid pSCW576.

### G. Conversion of pSCW576 Phagemid to A Single Strand Phagemid

The phagemid pSCW576 was grown up in the E.coli strain SCW105 and tested for its ability to produce single strand DNA.

The presence of single-strand DNA was determined by a complementary hybridization assay. Single strand templates were prepared from pSCW576 and the Genescribe phagemids, pTZ18R and pTZ18U using the procedures of IX.C. The latter two phagemids were chosen for tests because they have f1 intergenic regions in opposite orientations.

The complementary hybridization assay was performed as follows. Pairs of ss phagemid samples (pTZ18R, pTZ18U and pSCW576) to be tested for the ability to hybridize to each other were prepared by adding 0.5 μg of each ss phagemid in 50 μL of 20 mM Tris, pH 8.0, 20 mM EDTA and 0.2% SDS. Single phagemid samples are prepared as 0.5 μg of DNA in 25 μl of the above buffer. Each sample was then heated for 65°C for 1 hour. Hybridization was promoted by maintaining each sample at a temperature of 25°C over 3 hours. Then a Tris-borate 1% agarose gel was run with the hybridized samples according to Maniatis except the gel contained 1 μg ethidium-bromide/mL.

Plasmid sample pairs that hybridize together result in larger molecular weight bands than the individual DNA samples. It was observed that pSCW576 hybridized to only one of the pTZ18 phagemids. This was taken as proof that BamHI mobile f1 in pSCW576 functioned to convert a plasmid to a phagemid since f'(IG) pSCW576 could only be in one orientation relative to pTZ18R or pTZ18U. Thus the addition of BamHI mobile f1 to pSCW231 caused the conversion thereof into phagemid pSCW576.

### IX. Standard Procedures

The procedures and reagents in this section were used frequently in the methods presented supra, for the preparation of mobile f1 and phagemids containing mobile f1.

### A. Competent E.coli Cell Preparation

This is a procedure for preparing E.coli to accept plasmids.

Inoculate the desired E.coli strain into 5 μL of LB medium and grow overnight with shaking at 37°C. The next day dilute 0.5 ml of the E.coli culture into 50 mL of LB medium and grow to a cell density of 80 Klett units (Red Filter) at 37°C with shaking.

Centrifuge cells at 5,000g for 10 minutes and resuspend cell pellet in 20 mls of 4°C 0.05M CaCl₂ and set on ice for 30 minutes. Centrifuge cells at 5,000g for 10 minutes and resuspend cell pellet in 10 mL of 0.05M CaCl₂.

To make frozen competent cells for transformation add 10 mls of 40% glycerol to the resuspended cell pellet, mix well and freeze 0.5 mL aliquots in tubes of competent E.coli cell suspension at -70°C. These are good for at least three months.

B. Plasmid Transformation of Competent E.coli

To 0.5 mL of (thawed) competent E.coli cells add 0.05 to 1 µg of plasmid DNA or DNA ligation mix and set on ice for 1 hour. Heat shock the transformation tube of competent cells at 42°C for 2 minutes. Maintain the cells at 37°C for 10 minutes. Transfer the cells to a 15 mL tube. Add 1 mL of LB medium to the tube and shake at 37°C for 1 hour to nonselectively grow an E.coli cell suspension. Spread a 200 µL amount of the nonselective cell suspension and 200 µL amounts of $10^{-1}$, $10^{-2}$ and $10^{-3}$ suspension of dilutions in LB media onto separate antibiotic selective LB plates. Antibiotics are used at 100 µg/mL ampicillin (a penicillin analogue), chlorampenicol at 35 µg/mL and kanamycin at 30 µg/mL. The plates are grown overnight at 37°C and single colony transfectants are picked for further analysis.

C. Preparation of Single Strand Template DNA

Inoculate 5 ml of LB medium containing appropriate antibiotic marker for both plasmid and F' with the strain containing the desired plasmid clone and incubate while shaking overnight at 37°C. Use a fresh single colony or a glycerol stock to inoculate the culture used to make single-stranded DNA.

Using 1 mL of this overnight culture, inoculate 50 mL of 2XYT (E. coli growth media) contained in a 250 mL flask. Incubate 30 minutes at 37°C with vigorous agitation. This culture also contains ampicillin (100 µg/mL) and 35 µg/mL chlorampenicol. Infect the culture by adding 10 µL of a R408 phage stock of $10^{10}$ phage/ml. Continue shaking at 37°C for 14 to 18 hours or overnight.

Centrifuge the culture 15 minutes, 17,000 g at 4°C and retain the supernatant which contains the ss phagemid. Centrifuge again to remove all cells.

Measure the volume of the supernatant in a graduated cylinder. Add 1/4 volume of solution containing 20% polyethyleneglycol (PEG 8,000) and 3.5M ammonium acetate. Mix gently and store on ice 30 minutes. Centrifuge 15 minutes at 17,000xg and 4°C to collect the precipitated phage particles.

Drain the supernatant and resuspend the pellet in 0.5 mL of TE buffer. Transfer suspension to a 1.5 mL centrifuge tube. Extract by adding 0.5 mL of Tris-EDTA buffered phenol. Mix on a vortex mixer 1 full minute. Then centrifuge 5 minutes to separate the phases. Transfer the top aqueous phase to a fresh tube, being careful to leave behind any interface material.

Repeat this extraction five or six times until the amount of interface material diminishes so that none is seen in the final extraction. Extract the final aqueous phase with 0.5 ml of a isoamylalcohol:chloroform mixture (1:24). Repeat 2 times to remove last traces of phenol.

Precipitate the ssDNA by adding NaCl to 0.15 mM and 2.5 volumes EtOH in 100% ethanol. Chill to -70°C (dry ice/ethanol bath) for 30 minutes. Centrifuge 15 minutes at -20°C.

Drain the supernatant carefully and wash the essentially invisible pellet with 95% ethanol. Dry the pellet (air or vacuum). Dissolve the pellet in 20 µL of distilled water. The ssDNA should be stored in a fresh tube at -20°C. Yield is about 5-6 µg of single strand DNA at 50 to 95% purity.

D. Large Scale Plasmid Preparation (Large Batches)

Inoculate the desired E.coli strain into 10 mL of LB medium and 10g NaCl in 1 L. Grow E.coli by shaking culture overnight at 37°C. Inoculate the overnight culture into 800 mL LB medium. Shake medium overnight at 37°C for at least 20 hours. Centrifuge cells in Sorvall GS3 rotor (2 bottles per culture) 5,000 g for 7 minutes at 4°C. Pour off supernatant.

Resuspend resulting pellet in 20 mL of a solution containing 50 mM glucose, 10 mM EDTA and 25 mM Tris-Cl (pH 8). Transfer cells to GSA bottles and let set for 5 minutes. Add 40 mL of freshly prepared solution that is 0.2N NaOH and 1% SDS. Gently invert the bottle a few times to mix. Store on ice 10 minutes.

Add 30 mL of a solution containing 60 mL of 5M potassium acetate, 11.5 mL glacial acetic acid and 28.5 ml H₂O. Invert several times to mix. Set on ice for 15 minutes. Centrifuge in Sorvall GSA rotor with inserts at 10,000 g for 15 minutes at 4°C. Pour supernatant into a clean GSA bottle.

Measure supernatant volume. Add an equal volume of isopropanol. Mix and let sit on ice 15 to 30 minutes. Centrifuge at 9.5K with inserts in GSA rotor for 20 minutes at 4°C. Pour off supernatant and wash resulting pellet with isopropanol. Dry pellet and resuspend in 6 mL of TE. Add 100 µL of 10 mg/mL RNAse A solution. Heat solution at 37°C for 30 minutes. Extract solution 3 times with equal volumes of phenol (or until interphase is clear). Then extract with equal volumes of a chloroform/isoamyl alcohol mixture (24:1) 2 times or until interphase is clear.

Add NaCl to make 0.15M NaCl concentration. Add equal volume of isopropanol. Bathe in dry ice and ethanol for 15 minutes. Centrifuge 10,000 g in mL polypropylene tubes 15 minutes at 4°C. Pour off supernatant. Wash pellet in 70% ethanol. Dry pellet briefly. Resuspend in 2 mls H₂0. Add 800 µL PEG 8000 solution. PEG 8000 solution is 30% PEG 8000 in 1.8 M NaCl. Aliquot about 1.4 ml of solution into 1.5 ml microcentrifuge tube. Let sample sit at least 16 hours at 4°C. Centrifuge in 15 ml tubes at 15,000 g for 30 minutes with rubber adapters. Pour off supernatant. Quick rinse with 70% ethanol to remove residual PEG. Pellet will appear glassy. Resuspend in 400 µL H₂0. Add 13 µL of 3M Sodium Acetate (ph 5.5) (final concentration 100 mM) and 800 µl EtOH. Let sit at room temperature for 5 minutes. Centrifuge in 15 ml tubes at 15K for 30 minutes at 4°C. Wash pellet with 70% ethanol.

Resuspend pellet in desired volume of TE of about 1 ml.

E. Mini DNA Preparative Procedure (Small Batches)

An E.coli transfectant containing a plasmid is inoculated into 5 ml of LB containing the appropriate selective antibiotic for the plasmid. The transfectant is grown overnight in a roller drum at 37°C. Two mL of the saturated overnight culture is centrifuged at 10,000 g's for 20 seconds in a room temperature microcentrifuge. The pellet of cells is resuspended in 100 μL containing 50 mM glucose, 25 mM Tris pH 8.0, 10 mM EDTA. The suspension set for 5 minutes at room temperature (22-25°C). To dissolve the cells add, 200 μL of a solution that is 0.2N NaOH and 1% SDS (made fresh) and mix gently by inversion. Set the mixture on ice for 5 minutes. To precipitate cell debris, add 150 μL of 4°C 5M potassium acetate (pH 4.8). Mix gently and set for 5 minutes on ice. To remove cell debris, centrifuge for 2 minutes at 10,000g in a room temperature microcentrifuge. Remove the supernatant. Add 100 μL of TE. Extract once with 0.5 mL of phenol saturated with TE buffer and once with 0.5 mL of isoamyl alcohol:chloroform mix (1:24). Precipitate the DNA by bringing the concentration of the extracted supernatant to 150 mM NaCl. Then add 2.5 volumes of 100% ethanol thereto. Set mixture on a dry ice/ethanol bath of about -70°C for 30 minutes. To collect miniprep plasmid DNA, centrifuge for 5 minutes in a -20°C microcentrifuge. Dry the DNA at room temperature briefly. Resuspend DNA in 100 μL of TE (about 1-2 μg).

F. Gel Fragment Isolation

Visualize band of interest with ultraviolet lamp. Remove ethidium bromide labeled DNA fragment by excising gel with scalpel. Rinse dialysis tubing with $H_2O$ several times. Then rinse tubing with 1% gelatin. Rinse several times with $H_2O$. Fill dialysis bag with tris acetate EDTA electrophoresis buffer (TAE). Drop excised gel into bag. Squeeze out excess TAE from bag, leaving just enough to cover gel piece. Lay the bag flat and perpendicular to the electrodes of a horizontal electrophoresis apparatus filled with 1X TAE buffer. Apply 150V until all traces of ethidium bromide labeled DNA have migrated off the gel into the bag. Reverse current 1 minute at 150V to back DNA off the sides of the tubing. Mix solution back and forth and empty into a tube. Rinse bag with minimal amount of $H_2O$ and add to tube. Precipitate DNA by adding NaCl to 0.15M. Add 2X volume ethanol. Place tube in -70°C dry ice/ethanol bath for 1 hour. Centrifuge at -20°C in a 15 ml tube at 15,000 g for 30 minutes. Wash pellet with 70% ethanol. Dry pellet briefly. Resuspend pellet to desired volume with TE.

G. Reagent Abbreviations and Preparations

EDTA - (ethylenediaminetetraacetic acid, di sodium salt) (Sigma)
ATP - adenine triphosphate (Pharmacia)
DTT - dithiothreitol (Eastman Kodak Company)
TE - 10 mM Tris, 1 mM EDTA, pH 8.0 (Sigma)
BSA - bovine serum albumin, pentax V fraction (Miles)
dATP - deoxy adenine triphosphate
dCTP - deoxy cytosine triphosphate
dGTP - deoxy guanine triphosphate
dTTP - deoxy thymidine triphosphate
SDS - sodium dodecyl sulfate (BDH Chemicals)
EtOH - 100% ethanol
TAE - Trisacetate EDTA buffer prepared according to Maniatis, supra.
Tris - Tris(hydroxymethyl)aminomethane
LB Media - To prepare LB media add 10g Bactotryptone (Difco), 5g yeast extract (Difco) and 10g NaCl (Eastman Kodak Company) to 1 liter of nanopure water (Millipore). To prepare solid LB media for plates add 20g of Bacto-agar (Difco) to the above liquid media.

**Claims**

1. A f1 phage intergenic region having the same synthetic restriction site on each of its flanks.

2. The f1 phage intergenic region of claim 1 comprising 454 base pairs.

3. The f1 phage intergenic region of claim 1 comprising sufficient base pairs to cause a plasmid to become single-stranded.

4. The f1 phage intergenic region of claim 1, 2 or 3 wherein the restriction site is selected from the group consisting of BamHI, XbaI, SalI, BglII, XhoI, SpeI, AvrII and NheI.

5. The f1 phage intergenic region of claim 1, 2 or 3 wherein the restriction sites form cohesive ends.

6. A phagemid comprising an f1 phage intergenic region having on each flank of said region synthetic DNA sequences formed from the fusion of two different restriction sites.

7. The phagemid of claim 6 wherein the flanking DNA sequences are formed from pairs of restriction DNA endonuclease sequence selected from the group consisting of:
    a) BamI and BclI;
    b) BamHI and BglII;
    c) BamHI and XhoII;
    d) XbaI and SpeI;
    e) XbaI and AVRII; and
    f) XbaI and NheI.

8. A protein expression phagemid having the f1 phage intergenic region and flanking DNA sequences of claim 6 or 7.

9. A retrovirus phagemid having the f1 phage intergenic region and flanking DNA sequences of claim 6 or 7.

10. A transfection phagemid having the f1 phage intergenic region and flanking DNA sequences of claim 6 or 7.

FIG. I

EP 0 356 130 A2

EP 0 356 130 A2

```
          +1
          10         20         30         40         50         60         70         80         90        100
First nt.  |          |          |          |          |          |          |          |          |          |
  +1     GATCCTGTA AACGTTAATA TTTTGTTAAA ATTCGCGTTA AATTTTTGTT AAATCAGCTC ATTTTTTAAC CAATAGGCCG AAATCGGCAA AATCCCTTAT
           GACAT TTGCAATTAT AAAACAATTT TAAGCGCAAT TTAAAAACAA TTTAGTCGAG TAAAAAATTG GTTATCCGGC TTTAGCCGTT TTAGGGAATA

  +101    AAATCAAAAG AATAGACCGA GATAGGGTTG AGTGTTGTTC CAGTTTGGAA CAAGAGTCCA CTATTAAAGA ACGTGGACTC CAACGTCAAA GGGCGAAAAA
           TTTAGTTTTC TTATCTGGCT CTATCCCAAC TCACAACAAG GTCAAACCTT GTTCTCAGGT GATAATTTCT TGCACCTGAG GTTGCAGTTT CCCGCTTTTT

  +201    CCGTCTATCA GGGCGATGGC CCACTACGTG AACCATCACC CTAATCAAGT TTTTTGGGGT CGAGGTGCCG TAAAGCACTA AATCGGAACC CTAAAGGGAG
           GGCAGATAGT CCCGCTACCG GGTGATGCAC TTGGTAGTGG GATTAGTTCA AAAAACCCCA GCTCCACGGC ATTTCGTGAT TTAGCCTTGG GATTTCCCTC

  +301    CCCCCGATTT AGAGCTTGAC GGGGAAAGCC GGCGAACGTG GCGAGAAAGG AAGGGAAGAA AGCGAAAGGA GCGGGCGCTA GGGCGCTGGC AAGTGTAGCG
           GGGGGCTAAA TCTCGAACTG CCCCTTTCGG CCGCTTGCAC CGCTCTTTCC TTCCCTTCTT TCGCTTTCCT CGCCCGCGAT CCCGCGACCG TTCACATCGC

  +401    GTCACGCTGC GCGTAACCAC CACACCCGCC GCGCTTAATG CGCCGCTACA GGGCGCGTCA G
           CAGTGCGACG CGCATTGGTG GTGTGGGCGG CGCGAATTAC GCGGCGATGT CCCGCGCAGT CCTAG
```

FIG. 2

MOBILE f₁

2 | SELECT PLASMID

SELECTED PLASMID

3 | IDENTIFY UNIQUE RESTRICTION SITE

UNIQUE RESTRICTION SITE

4 | OPEN PLASMID

COHESIVE ENDS

PLASMID BACKBONE

5 | BLUNT COHESIVE ENDS

6 | ADD RESTRICTION SEQUENCES

RESTRICTION SEQUENCES

7 | TREAT WITH RESTRICTION ENDONUCLEASE

COHESIVE ENDS

8 | SELF LIGATE

8

RECEIVING PLASMID

UNIQUE RECEIVING SITE

9 | TRANSFORMING E.COLI WITH RECEIVING PLASMID

10 → ISOLATING RECEIVING PLASMID

TREAT RECEIVING PLASMID WITH RESTRICTION ENDONUCLEASE

11

RECEIVING PLASMID

12 | LIGATE MOBILE f₁ WITH RECEIVING PLASMID BACKBONE

MOBILE f₁

PHAGEMID

FIG. 3